# EUROPEAN PATENT APPLICATION

(11) **EP 2 434 005 A1**
(43) Date of publication of application: **28.03.2012**
(21) Application number: 10010502.2
(22) Date of filing: 24.09.2010
(51) Int. Cl.: C12M 1/107

(54) **Method and device for the production of bio gas from a biomass feed stock**

(71) Applicant: Essent Energie Verkoop Nederland B.V., 5211 AK'S-Hertogenbosch (NL)
(72) Inventor: van Mechelen, Xander, 4813 BC Breda (NL); de Jong, Jan, 6076 NS Haaren (NL)
(74) Representative: Polypatent

(57) **Abstract**

The invention refers to a method for producing gas from biomass, in particular by digestion of manure in at least one fermentation device, wherein at least a part of the fermentation heat is recovered from a subsequent liquefaction of the gas in a coupled liquefaction device to the fermentation device.

## Description

The invention refers to a method for producing gas from biomass, in particular by digesting of manure. The invention moreover refers to a device for the production of gas from a biomass feed stock for producing such gas.

Production of hydrocarbons in the form of liquid or gaseous fuels, for instance in the form of methane by fermentation/digestion of biomasses such as animal waste in the form of manure or diluted manure in fermentation devices is generally known In the art. Such fermentation/digestion usually requires an anaerobic atmosphere, as well as a suitable temperature which is normally achieved by heat treatment of the biomass. Heat treatment in the absence of oxygen causes pyrolysis of the volatic constituents which results in a creation of gas comprising CO, H₂, CH₄. After removal of impurifications, a combustible gas is obtained which may be used as a fuel.

In livestock farming, often fermenters for the production of biogas are to be found. It is generally known to combine such fermentation devices with CHP technology (Combined Heat and Power). In biomass CHP systems, biogas is burned in a gas combustion engine which drives a generator for the production of electricity. The hot exhausted gases from the combustion engine are recovered in a heat recovery unit. The thermal energy is partly used for heating and hot water supply. However, the major amount of the thermal energy is lost/wasted.

Generally, manure fermenters are an effective solution to deploy the animal waste and at the same time to save electrical energy.

Only recently a new type of small scale manure fermenters have been introduced.

However, there is still potential for energy savings and for optimization. As mentioned before, fermentation of the biomass requires heating which partly consumes the energy produced with the CHP system.

It is, accordingly, an object of the present invention, to provide a method for producing gas from biomass which is more effective and less lossy compared to the know biomass CHP systems. In particular, it is an object of the present invention to provide an integrated energy system for a farmery which effectively uses the animal waste incurred at the farmery.

This and other objects are achieved by a method for producing gas from biomass, in particular by digestion of manure in at least one fermentation device, wherein at least a part of the heat required for fermentation is obtained/recovered by shifting thermal energy from a subsequent liquefaction of the gas in a coupled liquefaction device to the fermentation device.

In other words, the invention provides an advantageous combination of a small scale biogas liquefaction device with a small scale biogas fermentation device in a very cost-effective manner. Liquefied methane can be effectively stored, bottled and for instance used for fueling of cars, trucks and other vehicles. Methane will liquefy at temperatures of around -160°C at atmospheric pressure. The rejected heat from methane liquefaction may be almost completely used for the fermentation process.

In one embodiment of the method according to the invention, heat shifting may be conducted by utilizing at least one heat exchanger. It might be appropriate, depending on the temperature level of the rejected heat from the gas liquefaction, to conduct heat shifting by utilizing at least one heat pump.

In one advantageous embodiment of the method according to the present invention, additional thermal energy for fermentation is provided by shifting heat recovered from milk cooling into the fermentation device.

This may be achieved by utilizing rejected heat of a coupled milk cooling device, the heat being transferred to the fermentation device.

Alternatively, and/or additionally, frozen carbon dioxide generated by the gas liquefaction device may be utilized for cooling the milk. This may be achieved by evaporating the frozen carbon dioxide which will incur anyway during gas liquefaction.

As mentioned before, the liquefied biogas may be stored in an appropriate vessel prior to merchandising. Thereby unavoidably a certain amount of boil-off will be incurred.

In one embodiment of the method according to the invention, the gas boil-off from stored liquefied gas is burnt in a gas heater for generating thermal energy to be additionally provided to the fermentation reactor.

The aforementioned objects are also achieved by a device for the production of biogas from a biomass feed stock including at least one fermenting reactor, at least a gas liquefaction device coupled to the reactor and means for transfer of rejected heat from the liquefaction of gas into the reactor.

In one embodiment, the device includes at least one heat mixing device connected to the reactor and receiving thermal energy from the liquefaction device and at least from one further heat source. The heat mixing device may be connected to a gas heater in which for instance the aforementioned gas boil-off may be burnt. Additionally, low pressure gas from an external gas source may be burnt in the gas heater. The liquid gas boil-off may be one of the aforementioned further heat sources.

An appropriate other heat source may be a milk cooling device which is on a farmery anyway required to cool down milk from 35°C to about 4°C for transport and merchandising. The gas mixing device may be connected to a milk cooling device receiving rejected heat from the milk cooling device.

Advantageously, the device according to the invention is integrated into a dairy farm and fed with manure incurred at the farm.

Other advantages and features of the present invention may be readily apparent from the following specification and detailed description of one possible embodiment of the device according to the present invention. The embodiment will be described with reference to accompanying drawings in which:
- Figure 1: shows a block diagram of a combined fermenting and biogas liquefaction system implemented into a farmery,
- Figure 2: shows a block diagram explaining a combination of biogas liquefaction and milk cooling in a farmery, and
- Figure 3: shows another block diagram of the system according to Figure 1.

The method and device according to the invention are implemented into a cattle farm 1 with milk production where significant amounts of animal waste are incurred by the cattle. This biomass in the form of liquid and/or diluted manure is fed into a manure fermenter 2 in which anaerobic digestion of the manure takes place. Fermentation devices suitable for such small scale applications on a cattle farm are for instance disclosed in WO 2008/108644 A1 and WO 2007/061301 A1.

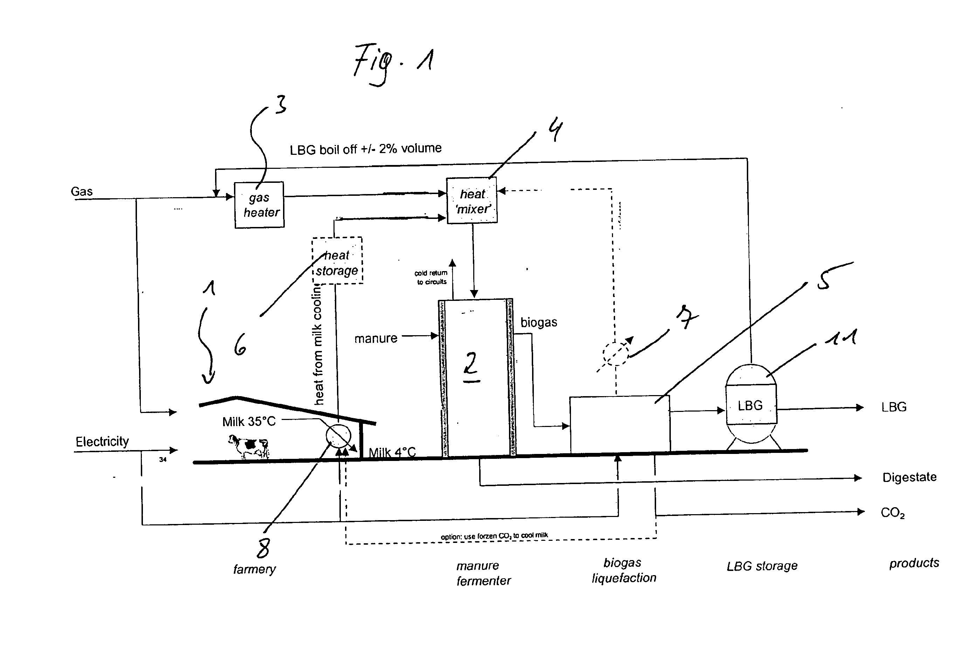

The fermenting process requires elevated temperatures within the manure fermenter 2. A part of the required heat is produced by a gas heater 3 as shown in figure 1.

Although the specific embodiment described herein refers to manure or diluted manure as biomass feed stock, it is to be understood that the manure fermenter 2 may be also fed with energy crops such as maiz silage or other biodegradable wastes incl. sewage, and food waste. During the process in the airtight manure fermenter 2, the biomass waste is converted into a gas mixture comprising CO, H₂, CH₄ and optionally other hydrocarbons as well as solid digestate which for instance may be used as a fertilizer.

The gas heater 3 is mainly fed from natural gas taken from a gas pipeline. Downstream the gas heater, a heat mixer 4 is provided which receives thermal energy from the gas heater 3 as well as from a small scale liquefaction device 5 and from a heat storage 6. The biogas liquefaction device 5 is fed by biogas which is produced within the manure fermenter 2. In the biogas liquefaction device 5, biogas is cooled down below its boiling point which is -161.6°C at atmospheric pressure. The rejected heat from biogas liquefaction is completely shifted into the manure fermenter 2 via a heat mixer 4.

In the embodiment shown in figure 1, additionally a heat pump 7 for transfer of thermal energy into the heat mixer 4 is provided. However, it is to be understood that the heat pump 7 is only optionally depending on the exit temperature of the rejected heat from the biogas liquefaction device 5.

As can also be taken from the embodiment in figure 1, the heat storage 6 is coupled to a milk cooling device 8 which is anyway required on the cattle farm 1 for cooling down the milk from approximately 35°C to approximately 4°C in which condition the milk is kept fresh and is transported to a dairy. The waste heat from the milk cooling device 8 is also transferred into the heat storage 6 and via heat mixer 4 into the manure fermenter 2.

Additionally, the milk may be cooled down by frozen carbon dioxide which will be incurred anyway during biogas liquefaction. The frozen carbon dioxide may be vaporized, thereby cooling down the milk which may be circulated in a heat exchanger as an heat transfer medium.

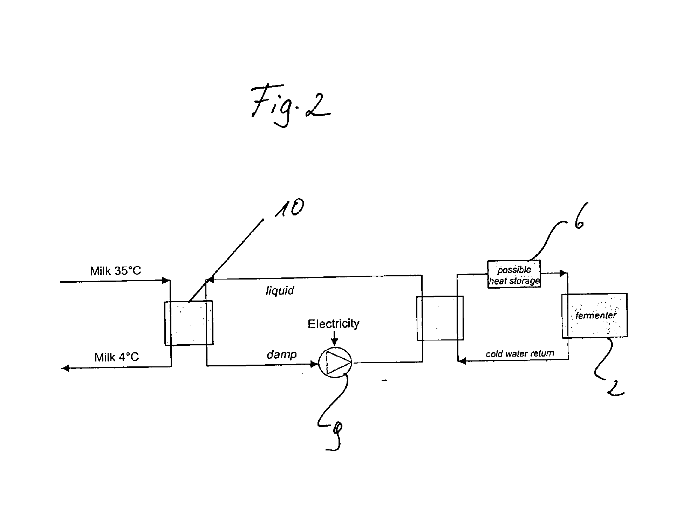

Figure 2 roughly outlines the principle of milk cooling and shifting the thermal energy from the milk to the manure fermenter 2. The milk cooling device 8 comprises a conventional cooling cycle with a heat transfer medium in a closed circuit which is firstly compressed by an electrically driven pump 9 and afterwards expanded and vaporized. Milk is circulated through an heat exchanger 10 in a rather conventional way. The milk cooling device 8 is coupled with a second heat exchanging circuit 10 through which water as an heat transfer medium circulates. The heat storage 6 as well as the manure fermenter 2 are part of this heat exchanging circuit.

The liquid biogas, in the drawings referred to as LBG, from the biogas liquefaction device 5 is stored in a liquid biogas tank 11, from which the biogas may be drawn for further transportation.

As this can also be taken from figure 1, the liquid biogas boil-off from the liquid biogas tank which might amount up to 2% in volume is fed to and burnt in the gas heater 3.

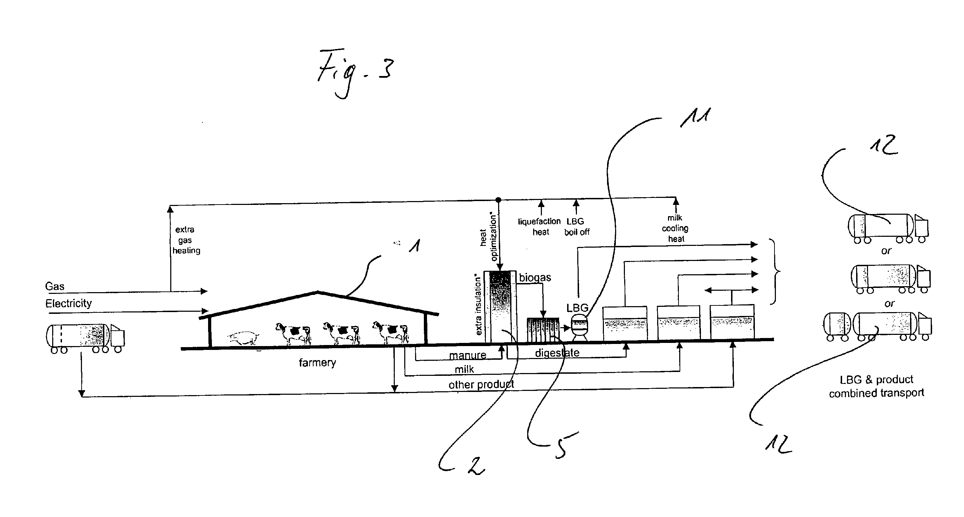

As this is outlined in figure 3, the method and device/system according to the invention includes an advantageous combination of handling and transport of a liquid biogas and another product, particularly milk.

Since on the cattle farm 1 milk will be collected by trucks 12 in regular time lags, handling and transportation of the liquid biogas may be conducted at the same time via the same transportation means.

### List of reference numerals

- 1: cattle farm
- 2: manure fermenter
- 3: gas heater
- 4: heat mixer
- 5: biogas liquefaction device
- 6: heat storage
- 7: heat pump
- 8: milk cooling device
- 9: pump
- 10: heat exchanger
- 11: liquid biogas tank
- 12: truck

## Claims

1. Method for producing gas from biomass, in particular by digestion of manure in at least one fermentation device, wherein at least a part of the fermentation heat is recovered by shifting thermal energy from a subsequent liquefaction of the gas in a coupled liquefaction device to said fermentation device.

2. Method according to claim 1, **characterized in that** heat shifting is conducted by utilizing at least one heat exchanger.

3. Method according to anyone of claims 1 or 2, **characterized in that** heat shifting is conducted by utilizing at least one heat pump.

4. Method according to anyone of claims 1 to 3, **characterized in that** additional thermal energy for fermentation is provided by shifting heat recovered from milk cooling into said fermentation device.

5. Method according to claim 4, **characterized by** utilizing rejected heat of a coupled milk cooling device, the heat being transferred to the fermentation device.

6. Method according to anyone of claims 1 to 4, **characterized by** utilization of frozen carbon dioxide generated by the gas liquefaction device for cooling of milk.

7. Method according to anyone of the preceding claims, **characterized in that** gas boil-off from stored liquefied gas is burnt in a gas heater for generating thermal energy to be provided to the fermentation device.

8. Device for the production of biogas from a biomass feed stock for conducting the method as specified in anyone of claims 1 to 7, including at least one fermentation reactor, at least a gas liquefaction device (5)
coupled to the reactor and means for transfer of rejected heat from liquefaction of gas into the reactor.

9. Device according to claim 8, including at least one heat mixing device (4) connected to the reactor and receiving thermal energy from the liquefaction device (5) and at least from one further heat source.

10. Device according to claim 9, **characterized in that** the heat mixing device (4) is connected to a gas heater (3).

11. Device according to anyone of the claims 9 or 10, **characterized In that** the heat mixing device (4) is connected to a milk cooling device (8) receiving rejected heat from the milk cooling device (8).

12. Use of a device according to anyone of the claims 8 to 11 on a dairy farm.
